# EUROPÄISCHE PATENTSCHRIFT

(11) **EP 2 326 915 B1**
(45) Veröffentlichungstag und Bekanntmachung des Hinweises auf die Patenterteilung: **15.04.2015**
(21) Anmeldenummer: 09782933.7
(22) Anmeldetag: 11.09.2009
(51) Int. Cl.: G01B 11/25, A61B 5/00, A61B 5/107

(54) **VERFAHREN UND ANORDNUNG ZUR ERFASSUNG VON KONTURDATEN UND/ODER OPTISCHEN EIGENSCHAFTEN EINES DREIDIMENSIONALEN SEMITRANSPARENTEN OBJEKTS**
METHOD AND APPARATUS FOR DETECTING CONTOUR DATA AND/OR OPTICAL CHARACTERISTICS OF A THREE-DIMENSIONAL SEMITRANSPARENT OBJECT
PROCÉDÉ ET DISPOSITIF DE DÉTECTION DE DONNÉES DE CONTOUR ET/OU DE PROPRIÉTÉS OPTIQUES D'UN OBJET SEMI-TRANSPARENT TRIDIMENSIONNEL

(30) Priorität: 12.09.2008 DE 102008044522
(43) Veröffentlichungstag der Anmeldung: 01.06.2011
(73) Patentinhaber: DeguDent GmbH, 63457 Hanau (DE)
(72) Erfinder: ERTL, Thomas, 61197 Florstadt (DE)
(74) Vertreter: Stoffregen, Hans-Herbert
(86) Internationale Anmeldenummer: PCT/EP2009/061827
(87) Internationale Veröffentlichungsnummer: WO 2010/029163

(56) Entgegenhaltungen:
- US-A- 5 248 876
- US-A1- 2002 134 921
- US-A1- 2007 296 959
- US-B1- 6 697 164

## Beschreibung

Die Erfindung bezieht sich auf ein Verfahren zur Erfassung von Konturdaten eines dreidimensionalen Objekts, insbesondere eines semitransparenten Objekts, wie zahnmedizinischen Objekts.

Gegenstand der Erfindung ist auch eine Anordnung zur Erfassung von Konturdaten eines dreidimensionalen Objektes, insbesondere eines semitransparenten Objektes wie zahnmedizinischen Objekts.

Ein Verfahren und eine Vorrichtung der eingangs genannten Art ist in der WO2006/048164 A2 beschrieben.

Bei dem bekannten Verfahren wird das Strahlenbündel vor Auftreten auf einen Strahlteiler in räumlich beabstandete parallele Einzellichtstrahlen aufgeteilt, wobei die Einzellichtstrahlen einen Abstand derart zueinander aufweisen, dass ein Auftreffen von reflektierten Einzellichtstrahlen auf unmittelbar aneinander grenzende Pixel des Bildsensors unterbleibt. Bei dem Verfahren wird eine Interferenz- und oder Autokorrelationsmessung eingesetzt, wobei in einem Strahlteiler aus dem Strahlenbündel ein Referenzstrahl abgespalten und von einem entlang des Referenzstrahls verschiebbaren Referenzspiegel reflektiert wird. Durch Verschieben des Referenzspiegels kann eine Position einer Signalgewinnungsfläche relativ zum Objekt festgelegt werden.

Aus der DE 699 28 453 T2 ist eine Vorrichtung zum Bestimmen einer Objektoberfläche eines Zahnbereichs bekannt. Die Vorrichtung umfasst eine Beleuchtungseinheit zum Bereitstellen einer Anordnung von Auflicht-Strahlen, welche zu dem Zahnbereich entlang einem optischen Pfad durch ein Sondenelement übertragen werden, um beleuchtete Punkte auf dem Bereich zu erzeugen. Ferner ist eine Licht fokussierende Optik vorgesehen, welche eine oder mehrere fokale Ebenen von der Abschlussfläche bei einer durch die Optik veränderbaren Position definiert, wobei jeder Lichtstrahl seinen Fokus auf einer der fokalen Ebenen hat. Ferner ist ein Translationsmechanismus vorgesehen zum Verlagern der fokalen Ebenen relativ zum Zahnbereich entlang einer Achse (Z), welche definiert ist durch die Ausbreitung der Auflicht-Strahlen.

Die von dem Zahnbereich reflektierte Strahlung wird von einem Detektor aufgenommen, der eine Anordnung von Erfassungselementen zum Messen der Intensität von jedem einer Mehrzahl von abbildenden Lichtstrahlen aufweist, welche von den Punkten zurückkehren und sich entlang einem optischen Pfad ausbreiten, welcher entgegengesetzt zu denen der Auflicht-Strahlen ist. Im Strahlengang ist zudem ein Spiegel angeordnet, welcher zwischen der Beleuchtungseinheit und der Licht fokussierenden Optik vorgesehen ist, wobei der Spiegel eine zentrale Apertur hat und die Auflicht-Strahlen in Richtung der Licht fokussierenden Optik leiten kann und die abbildenden Lichtstrahlen zu einem Detektor mittels einer Fläche des Spiegels, welches die Apertur umgibt, reflektieren kann. Mit dem Detektor ist ein Prozessor zum Bestimmen für jeden Lichtstrahl einer punktspezifischen Position verbunden, welcher die Position der entsprechenden Fokalebene der einen oder mehreren Fokalebenen ist, welcher die maximale gemessene Intensität des zurückgekehrten Lichtstrahls ergeben, und zum Erzeugen basierend auf den punktspezifischen Positionen, von Daten, welche repräsentativ für die Topologie des Bereichs sind.

Den oben beschriebenen Verfahren ist gemeinsam, dass vorzugsweise Licht aus einer bestimmten Entfernung (Z-Tiefe) detektiert wird. Eine Messung des Oberflächenprofils bzw. Volumens wird durch Durchfahren der Z-Koordinate, d. h. der Tiefe, mittels einer beweglichen Optik erreicht.

Von CD- / DVD-Playern ist ebenfalls bekannt, dass sich eine Tiefenselektion auch über Optik mit hoher numerischer Apertur erreichen lässt, die schnell defokussiert, wenn man die Soll-Z-Tiefe verlässt.

Es werden sowohl Vollfeldbeleuchtungen mit strukturiertem Licht, wie in der EP 0968687 A2, als auch Multipunktbeleuchtungen verwendet, wie diese in der WO2006/048163 A2 sowie der DE 699 28 453 C2 bzw. der WO00/08415 A1 beschrieben sind.

Der Nachteil von Vollfeldbeleuchtungen ist ein hoher Hintergrundsignalpegel, der durch Streuung von Beleuchtungslicht innerhalb des semitransparenten Objekts hervorgerufen wird sowie ein hoher Bedarf an Beleuchtungsintensität.

Bei Multipunktanordnungen ist der Bedarf an Beleuchtungsintensität deutlich geringer, jedoch kann auch hier der Fall auftreten, dass das Signal-Rauschverhältnis nicht ausreicht, bzw. die Empfindlichkeit gegen Umgebungslicht vermindert werden muss.

Die EP-A-1 548 481 bezieht sich auf ein Konfokalmikroskop, das mit polarisiertem Licht arbeitet. Ein Übersprechen der Strahlen einzelner Strahlenbündel soll vermieden werden.

Gegenstand der DE-A-10 2005 043 443 ist ein Lumineszenzmikroskop, wobei Bereiche eines zu messenden Objekts in unterschiedliche Lumineszenzzustände angeregt werden.

Nach der DE-A-197 37 760 sollen dreidimensionale Strukturen mittels richtungsabhängig codierter zeitlich erfassbarer Wellenzüge erfasst werden. Hierzu gelangt das Prinzip der Laufzeitmessung zur Anwendung.

Der US-A-2002/0134921 ist ein konfokales Mikroskop zu entnehmen. Um ein cross talking von von verschiedenen Lichtquellen stammenden Strahlen zu vermeiden, werden diese moduliert, um sodann die detektierten Signale der entsprechenden Lichtquelle zuordnen zu können. Dabei besteht die Möglichkeit, dass bei einem großen Abstand der einzelnen Lichtquellen diese nach einem gewünschten Muster angeordnet und gleich moduliert werden.

Um die Kontur eines Objekts zu messen, gelangt nach der US-A-2007/0296959 ein konfokales Messsystem zur Anwendung. Dabei wird ein pinhole array benutzt, um auf das Objekt Bildpunkte abzubilden, die sodann von einem Detektor erfasst werden. Zur Erfassung eines Höhenprofils wird der optische Weg der Lichtstrahlen verändert.

Mit einem konfokalen Messverfahren wird nach der US-B-6,697,164 die Kontur von Zähnen ermittelt. Dabei können in einer oder mehreren Fokusebenen Bildpunkte auf das Objekt abgebildet werden, um sodann die Intensität der rückabgebildeten Strahlung zu messen.

Bei einem konfokalen Messprinzip nach der US-A-5,248,876 werden gleichzeitig verschiedene Bildpunkte in verschiedenen Ebenen eines zu messenden Objekts abgebildet.

Der vorliegenden Erfindung liegt die Aufgabe zu Grunde, ein Verfahren und eine Anordnung der eingangs genannten Art so weiterzubilden, dass eine Verbesserung des Signal-Rauschverhältnisses und eine Reduktion des Hintergrundsignals erreicht wird. Es sollen mit hoher Genauigkeit Konturdaten des Objekts wie zumindest eines Zahns oder eines Bereichs eines solchen erfasst werden.

Die Aufgabe wird gelöst durch ein Verfahren zur Erfassung von Konturdaten eines dreidimensionalen Objekts, insbesondere eines semitransparenten Objekts wie zahnmedizinischen Objekts, wobei mittels einer optischen Einrichtung, vorzugsweise für konfokale oder OCT- oder depth of focus - Strahlengänge, voneinander versetzte Raster von Beleuchtungspunkten von zumindest zwei Multipunktbeleuchtungen auf das Objekt projiziert und diese sodann auf einen Pixel aufweisenden Sensor rückprojiziert werden,
- wobei der Abstand zweier Pixel (PI1, PI2) kleiner ist als der Abstand (E) zwischen den auf den Sensor (S) rückprojizierten Beleuchtungspunkten eines Beleuchtungsrasters (BRA, BRB) und somit auch als der Abstand (D) zwischen den auf den Sensor (S) rückprojizierten, einander zugeordneten ersten und zweiten Beleuchtungspunkten (BPA, BPB) der zueinander versetzten Beleuchtungsraster (BRA, BRB), wobei die ersten Beleuchtungspunkte (BPA) von einer ersten und die zweiten Beleuchtungspunkte (BPB) von einer zweiten der zumindest zwei Multipunktbeleuchtungen (LAA, LAB) stammen,
- wobei die Strahlungen der Beleuchtungspunkte der Multipunktbeleuchtungen in ihren Intensitäten moduliert werden und eine frequenz- und/oder phasenselektive Detektion der auf den Sensor rückprojizierten, einander zugeordneten ersten und zweiten Beleuchtungspunkten erfolgt und zur Ermittlung der Konturdaten Unterschiede von Intensität und/oder Frequenz der Messsignale benachbarter Pixeldes Sensors ausgewertet werden.

Vorzugsweise wird in einer an sich bekannten Messanordnung (Punkterasterprojektion auf ein Objekt und Rückprojektion mittels konfokaler Optik mit Verfahren der Konfokalebene, oder ähnlichen Verfahren wie "Depth of Focus") jeder Punkt einer Multipunktbeleuchtung in seiner Intensität moduliert und es erfolgt eine frequenz- und ggf. phasenselektive Detektion, wobei durch die Modulation eine sich räumlich und zeitlich ändernde Intensitätsverteilung in unmittelbarer lokaler Umgebung des Messorts- bzw. -bereichs und damit des Sensorbereichs, auf den der Messort bzw. -bereich abgebildet wird, ergibt.

Vorzugsweise werden dazu mindestens zwei Beleuchtungsquellen oder Lichtpunktegitter, die auch eine Matrix-LCD- oder DLP (Digital Light Processing)-Anordnung einschließen, verwendet, wobei die optische Achse der Lichtpunktegitter kollinear oder leicht gewinkelt zueinander angeordnet werden können. Die Strahlungen der Lichtpunktegitter werden unterschiedlich, vorzugsweise mit veränderter Phasenlage moduliert.

Aus Gründen der Vereinfachung wird nachstehend der Begriff Beleuchtungsquelle auch als Synonym für Lichtpunktegitter, das eine Matrix LCD- oder DLP-Anordnung einschließt, verwendet.

Im Fall zweier Beleuchtungsquellen liefert Beleuchtungsquelle A ein Beleuchtungspunkteraster A und Beleuchtungsquelle B ein Beleuchtungspunkteraster B. Dabei sind die Raster A und B leicht gegeneinander versetzt, so dass ein Beleuchtungspunkt aus Raster A von seinem korrespondierenden Punkt aus Raster B nur eine geringe Entfernung aufweist, jedenfalls deutlich geringer als der Abstand zum nächsten Rasterpunkt des eigenen Punkteraster. Insbesondere kann der Abstand zwischen zwei Beleuchtungspunkten eines Rasters oder Gitters im Bereich zwischen 100 µm bis 300 µm, insbesondere 200 µm, und der Abstand einander zugeordneter Beleuchtungspunkte der von den Rastern bzw. Gittern, die auf das Objekt abgebildet werden, im Bereich von 5 µm bis 100 µm, vorzugsweise zwischen 10 µm und 30 µm liegen, sofern es sich um eine kollineare Anordnung handelt, also die Strahlen der zumindest zwei Lichtpunktegitter in Richtung des Objekts parallel zueinander verlaufen. Der Abstand ist der Abstand zwischen den Mittelpunkten der Beleuchtungspunkte in der Fokusebene der Multipunktbeleuchtung. Der Durchmesser eines scharf abgebildeten Beleuchtungspunktes sollte zwischen 5 µm und 40 µm liegen. Ferner ist der Abstand von einander zugeordneten Beleuchtungspunkten der abgebildeten Beleuchtungspunkteraster oder -gitter derart, dass in der Fokusebene eine Überlappung nicht erfolgt.

Grundsätzlich sollte dann, wenn die Beleuchtungspunkte scharf auf dem Objekt abgebildet sind, der Abstand der Mittelpunkte der Beleuchtungspunkte eines Rasters zumindest 3 mal größer, vorzugsweise 3 bis 15 mal größer als der Abstand zweier einander zugeordneter Bildpunkte sein.

Mit anderen Worten werden die Multipunktbeleuchtungen mit den von diesen erzeugten Beleuchtungspunkterastern derart zueinander abgebildet, dass ein von einem der Raster stammender Beleuchtungspunkt zu einem nächstliegenden und damit zugeordneten Beleuchtungspunkt des anderen Rasters einen Abstand aufweist, der zumindest 4-mal kleiner als der nächstliegende Abstand von benachbarten Beleuchtungspunkten eines jeden Beleuchtungspunkterasters ist.

Im kollinearen Fall werden bei Messung in der Fokusebene die Beleuchtungspunkte der Lichtquellen A und B an unterschiedlichen Orten (z.B. anderen Pixeln des Sensors) abgebildet. Falls jedoch defokussiert gemessen wird, werden die Beleuchtungspunkte unscharf und vergrößert auf das Objekt abgebildet und somit auch unscharf auf den Sensor rückabgebildet.

Dadurch erhalten benachbarte Pixel des Sensors jeweils auch Licht von der jeweils anderen Beleuchtungsquelle. Dadurch werden die Unterschiede (z. B. bei gegenphasiger Modulation) in den Signalen geringer. Ausgewertet wird der maximale Signalunterschied zwischen den benachbarten Pixeln des Sensors, als Indikator für die beste Fokussierung in einer bestimmten z-Distanz vom Sensor (Differentialmessung). Um den entsprechenden Abstandswert in Richtung der optischen Achse (z-Achse) zu ermitteln, wird die fokale Ebene, d. h., Fokusebene entlang der z-Achse relativ zum Objekt verfahren. Dies erfolgt insbesondere durch Verstellen der Optik, über die die Beleuchtungspunkterraster bzw. -gitter und/oder die Messstrahlen auf den Sensor abgebildet werden.

Gemäß einer weiteren Ausführungsform ist die Messung mit nur einem zugeordneten Pixel möglich. Der eine eng benachbarte Beleuchtungspunkt wird bei Messung in der Fokusebene "ins Leere" abgebildet, während der andere direkt auf einen Pixel trifft. Im defokussierten Fall überlagert der jetzt größere Durchmesser des ursprünglich "ins Leere" laufenden Beleuchtungspunkt den anderen Beleuchtungspunkt und vermindert bei beispielsweise gegenphasiger Modulation das Signal. Dies kann notwendig sein bei Sensoren mit einem Pixelabstand, der deutlich größer ist, als der Abstand zwischen den Beleuchtungspunkten der Beleuchtungsraster A und B, also der einander zugeordneten Beleuchtungspunkte.

Im Fall von leicht zueinander gewinkelt angeordneten Beleuchtungsquellen kann ein Beleuchtungsstrahl koaxial zur Messrichtung laufen, der andere kreuzt den ersten in der Fokusebene oder beide Beleuchtungsstrahlen laufen leicht gewinkelt zur Messrichtung.

In diesem Fall kann die Beleuchtungsoptik auch nur schwach fokussierend oder gar nicht fokussierend ausgelegt sein, da neben der Aufweitung durch Defokussierung sich auch die räumliche Lage zumindest eines der beiden Beleuchtungspunkte in Abhängigkeit vom Z-Abstand ändert.

Es bestehen mehrere Auswertungsmöglichkeiten:
1. Im Falle von zwei Lichtquellen wird vorzugsweise mit der halben Frequenz der geplanten Detektionsfrequenz und vorzugsweise 180° Phasenversatz beleuchtet. In der Fokusebene überlagern sich beide Beleuchtungsstrahlen und erzeugen die Detektionsfrequenz mit entsprechender Phasenlage.
   Entsprechendes gilt für mehrere Lichtquellen z. B. 1/3 Frequenz und 120° Phasenversatz für 3 Lichtquellen etc.
   Außerhalb der Fokusebene laufen die Beleuchtungsstrahlen auseinander und die Intensität der Detektionsfrequenz wird geringer.
2. Bei beispielsweise zwei sich kreuzenden Strahlen kann auch festgestellt werden, ob sich die zu messende Oberfläche oberhalb oder unterhalb der Fokusebene befindet, da sich die Strahlen in der Fokusebene kreuzen und sich somit die Phasenlage am Ort des jeweiligen Messpixels (bezogen auf ein Nachbarpixel) beim Durchlaufen der Focusebene ändert. Dazu wird ein Modulationssignal z. B. + 90° zum Referenztakt und das andere -90° zum Referenztakt moduliert.

Gemäß einer weiteren bevorzugten Ausführungsform können vorzugsweise einzelne Beleuchtungspunkte oder Gruppen von Beleuchtungspunkten innerhalb des Beleuchtungsrasters mit unterschiedlicher Phasenlage und/oder Frequenz moduliert werden, um das Übersprechen der Messpunkte untereinander zu verringern. Dies kann beispielsweise mit einem DLP (Digital Light Processing ® Verfahren der Firma Texas Instruments) oder mit einem LCoS (Liquid Cristal on Silicon) Display erfolgen. Man kann dann mit Hilfe eines Referenztaktes das Signal unterschiedlicher Rasterpunkte nach Frequenz und Phasenlage trennen.

Erfindungsgemäß nutzt man das sich beim Durchfahren der Fokus- oder Schnittpunktebene verändernde Übersprechen (cross talk) zwischen benachbarten Beleuchtungspunkten, um die Konturdaten des zu messenden Objektes zu ermitteln. Im Falle gegenphasiger Modulation der Strahlungen der Beleuchtungspunkte der zumindest zwei Beleuchtungspunktegitter wird der maximale Signalunterschied zwischen benachbarten Pixeln eines Sensors genutzt, in deren Bereichen jeweils ein vom Objekt abgebildeter Beleuchtungspunkt des jeweiligen Lichtpunktegitters auftrifft, wobei die Beleuchtungspunkte einander so zugeordnet sind, dass diese in der Fokusebene einen Abstand aufweisen, der erheblich kleiner als der Abstand von Beleuchtungspunkten eines jeden Lichtpunktegitters. Diese Messart gelangt insbesondere bei Lichtpunktegittern zur Anwendung, bei denen die Beleuchtungsstrahlen kollinear verlaufen. Treffen die Beleuchtungspunkte nicht in der Fokusebene auf das Objekt, so erfolgt eine Defokussierung mit der Folge, dass die Intensitäten der von dem Objekt auf den Sensor abgebildeten Beleuchtungspunkte sich teilweise überlagern, also ein Übersprechen erfolgt, so dass eine Intensitätsreduzierung eintritt, die mittels der Pixel ermittelt wird.

Es wird die lokale Kontrasterzeugung bzw. deren Änderung in eng benachbarten Bereichen der Pixel des Sensors genutzt.

Werden Beleuchtungsquellen bzw. Lichtpunktegitter mit Strahlen verwendet, die leicht gewinkelt zueinander angeordnet sind, so schneiden sich die nicht notwendigerweise fokussierten Strahlen (z. B. Laserstrahlung) der einander zugeordneten Beleuchtungspunkte in der Fokusebene bzw. in der als Fokusebene definierten Strahlenschnittpunktebene, so dass gleichfalls eine Signaländerung einzelner Pixel und/oder der Signalunterschied zwischen den einzelnen Pixeln zur Bestimmung des Abstands Z ausgewertet werden; denn im Schnittpunkt ist z. B. bei gegenphasig modulierten Beleuchtungsquellen bzw. Lichtpunktegittern, durch die das Beleuchtungspunkteraster bzw. -gitter auf das Objekt abgebildet wird, die Intensität Null oder nahezu Null, wenn die einander zugeordneten Beleuchtungspunkte scharf auf dem Objekt abgebildet sind, sofern die Strahlen fokussiert sind. Befindet sich die Fokusebene vor oder hinter der Objektoberfläche, so ergibt sich eine Intensität, die von der im Schnittpunkt abweicht.

Anstelle einer gegenphasigen Modulation kann auch eine Pulsmodulation erfolgen. Entsprechend wird die Frequenz bzw. Pulsfolge der auf die Pixel des Sensors auftreffenden Strahlung ausgewertet, um den Z-Abstand zu ermitteln, bei dem die Strahlen der einander zugeordneten Bildpunkte sich auf der Objektoberfläche schneiden.

Bei der gewinkelten Anordnung der Beleuchtungsquellen bzw. Lichtpunktegitter ist es nicht erforderlich, dass man die Defokussierung ausnutzt, da der Schnittpunkt der Strahlen und die sich hieraus ergebenden Signale zur Auswertung benutzt werden.

Auch zeichnet sich die Erfindung aus durch eine Anordnung zur Erfassung von Konturdaten eines dreidimensionalen Objektes, insbesondere eines semitransparenten Objektes wie zahnmedizinischen Objekts, umfassend zumindest zwei Multipunktbeleuchtungen, sowie einen aufweisenden Sensor, wobei zwischen den Multipunktbeleuchtungen und dem Objekt und diesem und dem Sensor eine optische Einrichtung, vorzugsweise für konfokale, OCT- oder depth of focus-Strahlengänge angeordnet wird, über die zum einen von den Multipunktbeleuchtungen voneinander versetzte Raster von Beleuchtungspunkten auf das Objekt projiziert und zum anderen die Beleuchtungspunkte auf den Sensor rückprojiziert werden, wobei der Abstand zweier Pixel kleiner ist als der Abstand zwischen den auf den Sensor rückprojizierten Beleuchtungspunkten eines Beleuchtungsrasters und somit auch als der Abstand zwischen den auf den Sensor rückprojizierten, einander zugeordneten ersten und zweiten Beleuchtungspunkten der zueinander versetzten Beleuchtungsraster, wobei die ersten Beleuchtungspunkte von einer ersten und die zweiten Beleuchtungspunkte von einer zweiten der zumindest zwei Multipunktbeleuchtungen stammen, die Anordnung weiterhin umfassend
- Mittel zur Intensitätsmodulation der Strahlungen der Beleuchtungspunkte der Multipunktbeleuchtungen und
- Mittel zur frequenz- und/oder phasenselektiven Detektion der auf den Sensorrückprojizierten, einander zugeordneten ersten und zweiten Beleuchtungspunkte ,
- wobei der Abstand der ersten und zweiten Beleuchtungspunkte relativ zum Pixelabstand so ist, dass deren frequenz- und/oder phasenselektive Detektion es erlaubt, aus einer Auswertung der Unterschiede von Intensität und/oder Frequenz der Messsignale benachbarter Pixel des Sensors Konturdaten zu ermitteln.

Dabei können die Multipunktbeleuchtungen bzw. die von diesen erzeugten Lichtpunktegitter kollinear angeordnet sein. Es besteht aber auch die Möglichkeit, dass die Multipunktbeleuchtungen derart bzw. die Lichtpunktegitter zueinander angeordnet sind, dass die die Bildpunkte bildenden Strahlen geneigt zueinander verlaufen. Unabhängig hiervon sollte Abstand zwischen den Bildpunkten in der Fokusebene eines jeden Bildpunkterasters zwischen 100 µm und 300 µm liegen, betrachtet zwischen Bildpunktmitte zu Bildpunktmitte, wobei insbesondere Abstand der Bildpunkte eines jeden Bildpunkterasters in der Fokusebene drei- bis fünfmal größer ist als Abstand zwischen benachbarter einander zugeordneter Bildpunkte der Bildpunkteraster in der Fokusebene.

Ferner kann vorgesehen sein, dass Abstand benachbarter Pixel des Sensors gleich oder größer als Abstand zweier einander zugeordneter Bildpunkte der Bildpunkteraster in der Fokusebene ist.

Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus der nachfolgenden Beschreibung und von der Zeichnung zu entnehmenden bevorzugten Ausführungsbeispielen.

Es zeigen:
- Fig. 1: einen prinzipiellen Aufbau einer Vorrichtung zur Erfassung von Konturdaten eines Objekts mit zumindest zwei Beleuchtungsquellen, die kollinear zueinander angeordnet sind,
- Fig. 2: eine schematische Darstellung des Pixelrasters eines Sensors mit Beleuchtungspunkten aus zwei Beleuchtungsrastern,
- Fig. 3: eine schematische Darstellung von Beleuchtungspunkten aus zwei Beleuchtungsrastern auf dem Pixelraster des Sensors bei kollinearer Anordnung und defokussierter Optik,
- Fig. 4: eine schematische Anordnung der Beleuchtungspunkte mit unterschiedlichem Winkel zur Messrichtung,
- Fig. 5 a)-c): schematische Darstellungen des Pixelrasters des Sensors in Seitenansicht, mit gewinkelter Projektion eines ersten Beleuchtungsrasters und axialer Projektion eines zweiten Beleuchtungsrasters,
- Fig. 6 a)-c): schematische Darstellungen des Pixelrasters des Sensors in Seitenansicht, mit gewinkelter Projektionsrichtung des ersten Beleuchtungsrasters und gewinkelter Projektionsrichtung des zweiten Beleuchtungsrasters,
- Fig. 7: ein Diagramm von Taktfrequenzen zur Modulation der Beleuchtungsraster sowie ein Überlagerungssignal,
- Fig. 8: ein prinzipieller Aufbau einer alternativen Vorrichtung zur Erfassung von Konturdaten eines Objekts und
- Fig. 9: einen schematischen Aufbau eines Modulators zur Erzeugung von Modulationssignalen für die Lichtquellen.

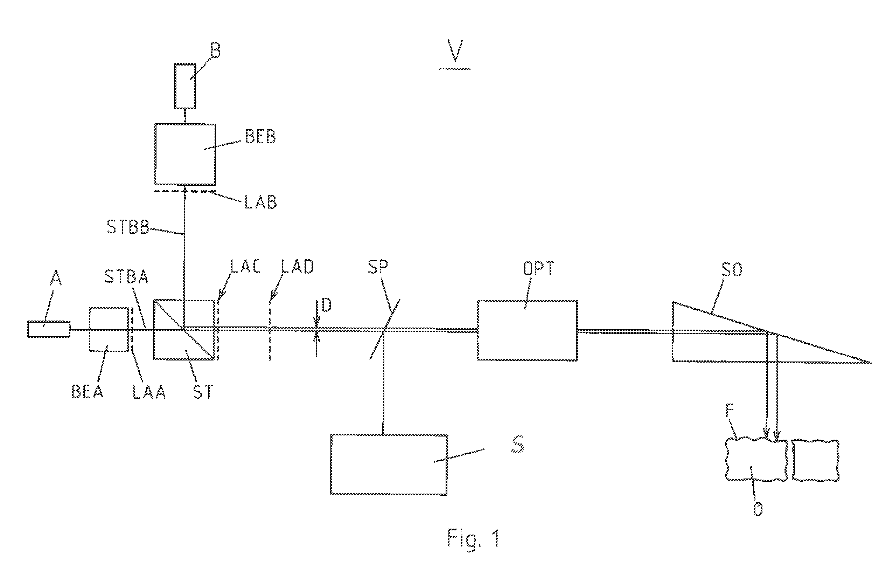

Fig. 1 zeigt einen schematischen Aufbau einer Vorrichtung V zur Erfassung von Konturdaten einer Freiformfläche F eines semitransparenten Objekts O wie Zahn.

Die Vorrichtung umfasst zwei Lichtquellen A, B wie Laserdioden, deren Licht jeweils über einen Beamexpander BEA, BEB aufgeweitet und jeweils auf ein Linsenarray LAA, LAB projiziert wird, wodurch ein Strahlenbündel STBA, STBB einer Vielzahl paralleler Einzelstrahlen erzeugt wird, so dass eine Punktrasterprojektion von ersten und zweiten Beleuchtungspunkten auf der Freifläche F des Objektes O erfolgt. Die Einzelstrahlen werden über einen Strahlteiler ST, einen teilweise durchlässigen Spiegel SP, eine vorzugsweise konfokale Optik OPT sowie ein endoskopisches Sondenelement SO auf das Objekt O geleitet, so dass auf diesem die ersten und zweiten Beleuchtungspunkte abgebildet werden. Die reflektierten Einzelstrahlen gelangen ebenfalls über das Sondenelement SO, die Optik OPT und den halbdurchlässigen Spiegel SP zu einem Detektor in Form eines Pixel PI aufweisenden Sensors S.

Alternativ zu den unmittelbar hinter den Beamexpandern BEA, BAB angeordneten Mikrolinsenarrays LAA, LAB kann ein einzelnes Mikrolinsenarray LAC als Verbund bzw. Einheit mit dem Strahlteiler ST ausgebildet sein. Eine weitere Alternative besteht darin, ein gemeinsames Mikrolinsenarray MLD separat dem Strahlteiler ST nachzuordnen.

Gemäß dem in Fig. 1 dargestellten Ausführungsbeispiel sind die Beleuchtungsquellen A, B mit dem Beamexpander BEA, BEB und den Linsenarrays LAA, LAB kollinear zueinander angeordnet und werden unterschiedlich, vorzugsweise mit veränderter Phasenlage moduliert.

Die Lichtquellen A, B mit den Beamexpandern BEA, BEB und den Linsenarrays LAA, LAB bilden dem Grunde nach ein Lichtpunktegitter, das technisch auch auf andere Weise realisiert werden kann. So kann z. B. eine LCD- oder DLP (Digital Light Processing)-Anordnung verwendet werden, mit der gleichfalls entsprechende Lichtpunkteraster auf dem Objekt O abgebildet werden, um die ersten und zweiten Beleuchtungspunkteraster oder -gitter zu erzeugen.

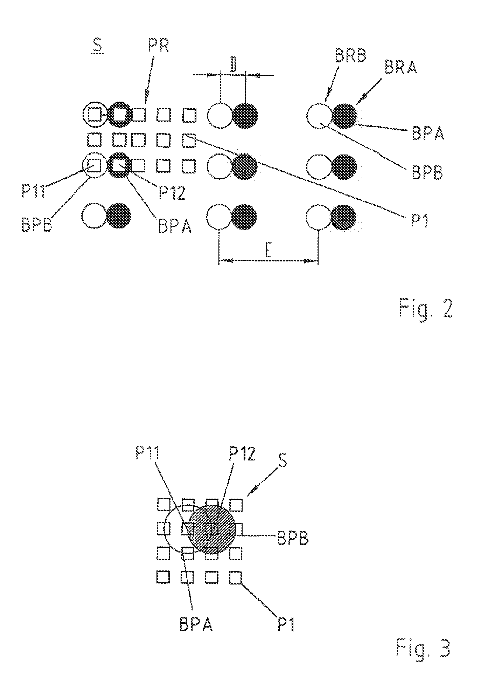

Im Fall zweier Beleuchtungsquellen A, B liefert Beleuchtungsquelle A ein Beleuchtungsraster BRA und Beleuchtungsquelle B ein Beleuchtungsraster BRB, wie dies in Fig. 2 dargestellt ist. Durch die kollineare Anordnung werden Beleuchtungsraster BRA, BRB parallel versetzt zueinander abgebildet. Die die Beleuchtungspunkte erzeugenden Strahlen verlaufen parallel zueinander. Jedes verlaufen parallel zueinander. Jedes Beleuchtungspunkteraster BRA, BRB besteht aus einzelnen Beleuchtungspunkten BPA sowie Beleuchtungspunkten BPB. Dabei sind die Beleuchtungsraster BRA, BRB um einen Abstand D leicht gegeneinander versetzt, so dass ein Beleuchtungspunkt BPA (erster Beleuchtungspunkt) des Beleuchtungsrasters BRA von seinem korrespondierenden, also zugeordneten Beleuchtungspunkt BPB (zweiter Beleuchtungspunkt) des Beleuchtungsrasters BRB nur eine geringe Entfernung aufweist. Diese Entfernung ist jedenfalls deutlich geringer als ein Abstand E zum nächsten Rasterpunkt des eigenen Punkterasters. Ferner zeigt Fig. 2 ein Pixelraster PR eines Sensors S bestehend aus einzelnen Pixeln P.

Insbesondere ist vorgesehen, dass der Abstand der jeweiligen Beleuchtungspunkte eines Beleuchtungsrasters zwischen 100 µm und 300 µm und der Abstand der einander zugeordneten Beleuchtungspunkte, also der ersten und zweiten Beleuchtungspunkte, zwischen 5 µm und 100 µm, vorzugsweise zwischen 10 µm und 30 µm liegt. Der Abstand ist dabei der Abstand zwischen Beleuchtungspunktmitte zu Beleuchtungspunktmitte in der Fokusebene.

Fig. 2 zeigt, dass im kollinearen Fall bei Messung in der Fokusebene die Beleuchtungspunkte BPA, BPB der Lichtquellen A und B an unterschiedlichen Orten, z. B. in anderen Pixeln PI1, PI2 abgebildet werden. Aus der Fig. 2 ergibt sich des Weiteren, dass dann, wenn die ersten und zweiten Beleuchtungspunkte, die einander zugeordnet sind, auf dem Objekt scharf abgebildet werden, also der zu messende Bereich des Objekts in der Fokusebene liegt, die Beleuchtungspunkte BPA, BPB sich nicht überlappen, sondern allerhöchstens berühren.

Falls jedoch defokussiert gemessen wird, wenn also der Bereich des Objekts O, auf den die einander zugeordneten ersten und zweiten Beleuchtungspunkte BPA, BPB abgebildet werden, nicht in der Fokusebene liegt, werden die Beleuchtungspunkte BPA, BPB unscharf und vergrößert auf das Objekt abgebildet und somit auch unscharf auf den Sensor S rückabgebildet. Dadurch erhalten benachbarte Pixel PI1, PI2 des Sensors S jeweils auch Licht von der jeweils anderen Lichtquelle A, B. Dadurch werden die Unterschiede, z. B. bei gegenphasiger Modulation, in den Signalen geringer (s. Fig. 3).

In diesem Fall wird der maximale Signalunterschied zwischen benachbarten Pixeln PI1, PI2 des Sensors S als Indikator für beste Fokussierung in einer bestimmten Z-Distanz zwischen Objekt O und Sensor S verwendet (Differentialmessung).

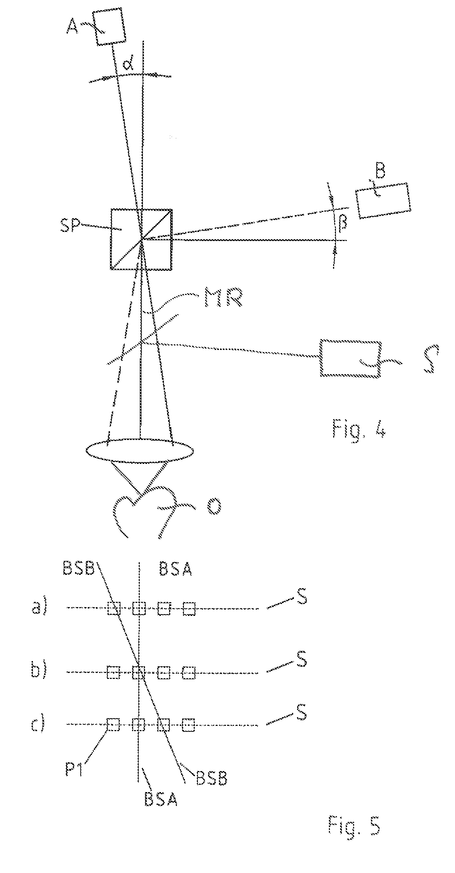

Fig. 4 zeigt eine Ausführungsform, bei der die Beleuchtungsquelle A in einem Winkel α zur Messrichtung MR und die Beleuchtungsquelle B in einem Winkel β zur Messrichtung MR angeordnet ist. Im Fall von leicht zueinander gewinkelt angeordneten Beleuchtungsquellen A, B kann ein Beleuchtungsstrahl BSA koaxial zur Messrichtung MR laufen, wobei der Beleuchtungsstrahl B der Lichtquelle B in einem Winkel zu dem Beleuchtungsstrahl A verläuft.

Alternativ können beide Beleuchtungsstrahlen BSA und BSB leicht gewinkelt zur Messrichtung MR verlaufen.

Die Fig. 5a) bis 5c) zeigen einen Blick auf den Sensor S von der Seite unter der Annahme, dass sich das zu messende Objekt O gemäß Fig. 5a) über der eingestellten Fokusebene befindet, gemäß Fig. 5b, in der eingestellten Fokusebene und gemäß Fig. 5c) bzw. unterhalb der eingestellten Fokusebene befindet.

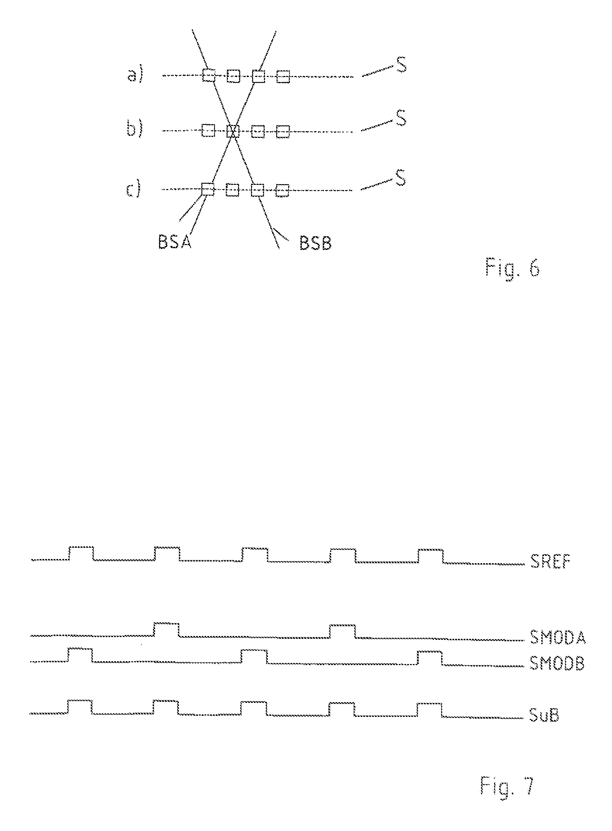

Die Fig. 6a) bis 6c) zeigen ebenfalls Pixelraster des Sensors S in Seitenansicht, wobei sich das Objekt gemäß Fig. 6a) oberhalb der Fokusebene, gem. Fig. 6b) innerhalb der Fokusebene und gemäß Fig. 6c) unterhalb der Fokusebene befindet.

In oben genannten Fällen kann die Beleuchtungsoptik auch nur schwach fokussierend oder gar nicht fokussierend ausgelegt sein, da neben der Aufweitung durch Defokussierung sich auch die räumliche Lage zumindest eines der beiden Beleuchtungspunkte in Abhängigkeit vom Z-Abstand ändert.

Aus den Fig. 5 und 6 wird erkennbar, dass dann, wenn die geneigt zueinander ausgerichteten Strahlen, die die ersten und zweiten Beleuchtungspunkte bilden, sich schneiden, Messort bzw. -punkt auf der Objektoberfläche F in der Fokusebene liegt. So ist die Anordnung ausgelegt. Dieser Schnittpunkt wird entsprechend auf den Sensor rückprojiziert. In diesem Fall wird das Messsignal des entsprechenden Pixels durch die beiden einander zugeordneten Beleuchtungspunkte bestimmt. Auf die unmittelbar benachbarten Pixel werden Beleuchtungspunkte nicht abgebildet. Infolgedessen wird gleichfalls die Messsignaldifferenz benachbarter Pixel ausgewertet, um eine Abstandsbestimmung (Z-Achse) zum Objekt durchzuführen.

Für diese Art der Auswertung ist Voraussetzung, dass der Abstand zweier Pixel kleiner als der Abstand zwischen den Bildpunkten eines Bildrasters ist und somit auch der Abstand zwischen einem Paar von zueinander angeordneten ersten und zweiten Bildpunkten. Ferner sollte die Anordnung optisch oder rechnerisch so ausgelegt werden, dass der Schnittpunkt zweier Strahlen, die die einander zugeordneten Beleuchtungspunkte erzeugen, in der Fokusebene des Sensors liegt.

Es sind mehrere Auswertungsmöglichkeiten möglich:
Im Falle von zwei Lichtquellen A, B bzw. Lichtpunktegittern, die z. B. auch durch LCDs oder DLPs gebildet werden, wird mit der halben Frequenz der geplanten Detektionsfrequenz und 180 ° Phasenversatz beleuchtet. In der Fokusebene gemäß Fig. 5b bzw. Fig. 6b überlagern sich beide Beleuchtungsstrahlen BSA, BSB und erzeugen die Detektionsfrequenz mit entsprechender Phasenlage, wie in Fig. 7 dargestellt. Fig. 7 zeigt das Referenzsignal SREF, ein Modulationssignal SMODA für Beleuchtungsraster BRA, ein Modulationssignal SMODB für das Beleuchtungsraster BRB sowie ein Überlagerungssignal SÜB.

Entsprechendes gilt für mehrere Lichtquellen z. B. ein Drittel Frequenz und 120 ° Phasenversatz für drei Lichtquellen usw. Außerhalb der Fokusebene laufen die Beleuchtungsstrahlen BSA, BSB auseinander und die Intensität der Detektionsfrequenz wird geringer (siehe Darstellung in Fig. 5a), c) sowie 6a), c)).

Eine weitere Auswertungsmöglichkeit besteht darin, bei beispielsweise zwei sich kreuzenden Beleuchtungsstrahlen BSA, BSB festzustellen, ob sich die zu messende Oberfläche che oberhalb oder unterhalb der Fokusebene befindet, da sich die Strahlen in der Fokusebene kreuzen und sich somit die Phasenlage am Ort des jeweiligen Messpixels PI, bezogen auf ein Nachbarpixel, beim Durchlaufen der Fokusebene ändert. Dazu wird ein Modulationssignal z. B. +90° zum Referenztakt und das andere -90° zum Referenztakt moduliert.

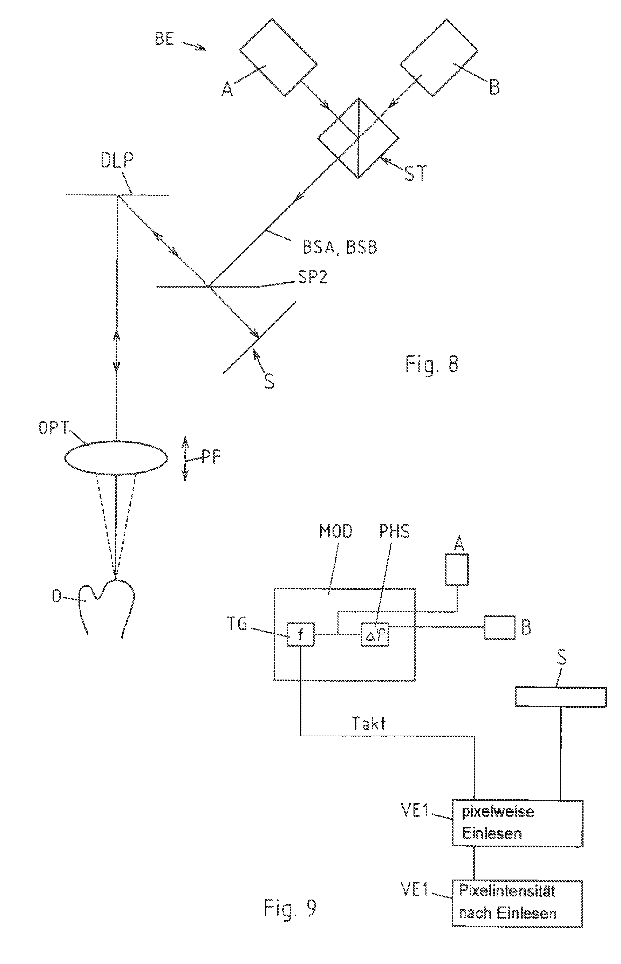

Weiterhin besteht die Möglichkeit, Gruppen von Beleuchtungspunkten bzw. einzelne Beleuchtungspunkte innerhalb eines Beleuchtungsrasters BRA, BRB mit unterschiedlicher Phasenlage oder Frequenz zu modulieren, um das Übersprechen der Messpunkte untereinander zu verringern. Dies kann beispielsweise mit einem DLP (Digital Light Processing - Verfahren der Firma Texas Instruments), oder mit einem LCoS (Liquid Cristal on Silicon) - Display erfolgen. In diesem Fall kann das Detektorsignal mit Hilfe eines Referenztaktes für die unterschiedlichen Rasterpunkte nach Frequenz und Phasenlage getrennt werden. Ein Ausführungsbeispiel ist in Fig. 8 schematisch dargestellt.

Fig. 8 zeigt eine Beleuchtungseinrichtung BE, wie diese in Bezug zu Fig. 1 bereits erläutert wurde. Die aus der Beleuchtungseinrichtung BE austretenden Beleuchtungsstrahlen BSA, BSB treffen auf einen halbdurchlässigen Spiegel SP2 und werden über ein DLP (Digital Light Processing) und eine verschiebbare Optik OPT auf das Objekt O abgebildet. Durch Verschieben der Optik entlang in Richtung der Pfeile PF kann die Fokusebene eingestellt werden. Die von dem Objekt O reflektierten Strahlen werden von dem Sensor S wie CMOS- oder CCD-Sensor erfasst.

Schließlich zeigt Fig. 9 einen Modulator MOD zur Modulation des aus der Lichtquelle A bzw. Lichtquelle B austretenden Lichtes. Dabei ist ein Ausgang eines Taktgenerators TG unmittelbar mit einem Steuereingang der Lichtquelle A und über einen Phasenshifter PHS mit einem Steuereingang der Lichtquelle B verbunden. Ein weiterer Ausgang des Taktgenerators TG ist mit einer Verarbeitungseinheit VE1 verbunden, in der die Pixel PI des Sensors S pixelweise eingelesen werden. Anschließend wird in einer weiteren Verarbeitungseinheit VE2 die Pixelintensität mit dem Takt verrechnet. Phasen und frequenzselektive Auswahl sind dabei möglich.

Erfindungsgemäß wird gezielt das Übersprechen, also cross talk genutzt, das von zwei einander zugeordneten Beleuchtungspunkten, die von vom dem Objekt O auf den Sensor S abgebildet werden, hervorgerufen werden kann. Dabei wird bei einer kollinearen Anordnung und gegenphasiger Modulation der Strahlung, die die ersten bzw. zweiten Beleuchtungspunkte erzeugen, maximale Intensitätsdifferenz benachbarter Pixel bzw. Pixelbereiche genutzt, über die von den rückprojizierten Beleuchtungspunkten Messsignale erzeugt werden.

Sofern die die Beleuchtungspunkte erzeugenden Strahlen geneigt zueinander verlaufen, können gleichfalls aus Abweichungen in der Intensität und/oder der Frequenz präzise Angaben über die Oberflächendaten des zu messenden Objektes gewonnen werden. Dabei muss die Eigenschaft des Defokussierens nicht zwingend genutzt werden, so dass z. B. auch eine Laserstrahlung verwendet werden kann, da die Schnittpunkte von den die Beleuchtungspunkte bildenden Strahlen zur Auswertung gelangen.

Ferner besteht die Möglichkeit, aus den Messdaten optische Eigenschaften wie wellenlängenabhängige Transparenz des Objektes abzuleiten, sofern eine spektrale Auswertung erfolgt.

## Patentansprüche

1. Verfahren zur Erfassung von Konturdaten eines dreidimensionalen Objekts (O), insbesondere eines semitransparenten Objekts wie zahnmedizinischen Objekts, wobei mittels einer optischen Einrichtung, vorzugsweise für konfokale oder OCT- oder depth of focus -Strahlengänge, voneinander versetzte Raster von Beleuchtungspunkten (BPA, BPB) von zumindest zwei Multipunktbeleuchtungen (LAA, LAB) auf das Objekt (O) projiziert und diese sodann auf einen Pixel (PI1, PI2) aufweisenden Sensor (S) rückprojiziert werden,
- wobei der Abstand zweier Pixel (PI1, PI2) kleiner ist als der Abstand (E) zwischen den auf den Sensor (S) rückprojizierten Beleuchtungspunkten eines Beleuchtungsrasters (BRA, BRB) und somit auch als der Abstand (D) zwischen den auf den Sensor (S) rückprojizierten, einander zugeordneten ersten und zweiten Beleuchtungspunkten (BPA, BPB) der zueinander versetzten Beleuchtungsraster (BRA, BRB), wobei die ersten Beleuchtungspunkte (BPA) von einer ersten und die zweiten Beleuchtungspunkte (BPB) von einer zweiten der zumindest zwei Multipunktbeleuchtungen (LAA, LAB) stammen,
- wobei die Strahlungen der Beleuchtungspunkte (BPA, BPB) der Multipunktbeleuchtungen (LAA, LAB) in ihren Intensitäten moduliert werden und eine frequenz- und/oder phasenselektive Detektion der auf den Sensor (S) rückprojizierten, einander zugeordneten ersten und zweiten Beleuchtungspunkten (BPA, BPB) erfolgt und
- zur Ermittlung der Konturdaten Unterschiede von Intensität und/oder Frequenz der Messsignale benachbarter Pixel (PI1, PI2) des Sensors (S) ausgewertet werden.

2. Verfahren nach Anspruch 1,
**dadurch gekennzeichnet,**
**dass** eine erste und eine zweite Multipunktbeleuchtung (LAA, LAB) verwendet werden, die jeweils auf das Objekt ein Beleuchtungspunkteraster (BRA, BRB) abbilden, bei denen der Abstand der einander zugeordneten ersten und zweiten Beleuchtungspunkte kleiner als der Abstand der ersten bzw. zweiten Beleuchtungspunkte (BPA, BPB) in dem jeweiligen Bildpunkteraster in der Fokusebene ist.

3. Verfahren nach Anspruch 1 oder Anspruch 2,
**dadurch gekennzeichnet,**
**dass** der Abstand zwischen benachbarten ersten Beleuchtungspunkten bzw. benachbarten zweiten Beleuchtungspunkten in der Fokusebene zumindest dreimal größer als der Abstand zwischen einem ersten Beleuchtungspunkt (BPA) und einem diesem zugeordneten zweiten Beleuchtungspunkt (BPB) gewählt wird.

4. Verfahren nach zumindest einem der vorhergehenden Ansprüche,
**dadurch gekennzeichnet,**
**dass** der Abstand benachbarter erster bzw. zweiter Beleuchtungspunkte (BPA, BPB) betrachtet zwischen deren Mittelpunkten in der Fokusebene so festgelegt wird, dass dieser in etwa zwischen 100 µm und 300 µm liegt.

5. Verfahren nach zumindest einem der vorhergehenden Ansprüche,
**dadurch gekennzeichnet,**
**dass** die Modulation der Strahlung bzw. Strahlungen und die Abbildung der Beleuchtungspunkte- (BPA, BPB) auf das Objekt (O) derart erfolgt, dass eine räumliche und/oder zeitlich ändernde Intensitätsverteilung in unmittelbarer lokaler Umgebung von zumindest einem Pixel (PI1, PI2) des Bildsensors (S) erfolgt.

6. Verfahren nach zumindest einem der vorhergehenden Ansprüche,
**dadurch gekennzeichnet,**
**dass** die Strahlungen der zumindest zwei Multipunktbeleuchtungen (LAA, LAB) gegenphasig moduliert werden und dass zur Konturbestimmung des Objekts (O) der maximale Signalunterschied zwischen benachbarten Pixeln (PI1, PI2) des Bildsensors (S) ausgewertet wird, in deren Bereich jeweils ein erster und ein diesem zugeordneter zweiter Beleuchtungspunkt (BPA, BPB) abgebildet werden.

7. Verfahren nach zumindest einem der vorhergehenden Ansprüche,
**dadurch gekennzeichnet,**
**dass** die zumindest zwei Multipunktbeleuchtungen (LAA, LAB) kollinear angeordnet werden.

8. Verfahren nach zumindest einem der vorhergehenden Ansprüche,
**dadurch gekennzeichnet,**
**dass** die die ersten und zweiten Beleuchtungspunkte (BPA, BPB) erzeugenden Strahlen der zumindest zwei Multipunktbeleuchtungen (LAA, LAB) geneigt bzw. gewinkelt zueinander verlaufen.

9. Verfahren nach Anspruch 8,
**dadurch gekennzeichnet,**
**dass** zumindest eine der zumindest zwei Multipunktbeleuchtungen (LAA, LAB) zur Messstrahlrichtung (MR) geneigt verläuft.

10. Verfahren nach Anspruch 8,
**dadurch gekennzeichnet,**
**dass** die zumindest zwei Multipunktbeleuchtungen (LAA, LAB) zur Messstrahlrichtung (MR) zueinander gewinkelt angeordnet werden.

11. Verfahren nach zumindest einem der vorhergehenden Ansprüche,
**dadurch gekennzeichnet,**
**dass** bei Multipunktbeleuchtungen (LAA, LAB), deren auf das Objekt (O) auftreffende Strahlen gewinkelt zueinander verlaufen, eine Optik verwendet wird, die schwach fokussierend oder nicht fokussierend ausgelegt ist.

12. Verfahren nach zumindest einem der vorhergehenden Ansprüche,
**dadurch gekennzeichnet,**
**dass** der Abstand zwischen einem Paar von einander zugeordneten ersten und zweiten Beleuchtungspunkten (BPA, BPB) zum Abstand der Pixel (PI1, PI2) des Sensors (S) derart gewählt wird, dass bei Messungen in der Fokusebene die ersten Beleuchtungspunkte (BPA) auf pixelfreie Bereiche des Bildsensors (S) und die zweiten Beleuchtungspunkte (BPB) auf ein Pixel (PM) des Bildsensors (S) treffen und dass bei Defokussieren sowohl die ersten als auch die zweiten Beleuchtungspunkte auf Pixel (PI1, PI2) treffen.

13. Verfahren nach zumindest einem der vorhergehenden Ansprüche,
**dadurch gekennzeichnet,**
**dass** bei der Verwendung von n-Lichtquellen zur Erzeugung von entsprechenden Multipunktbeleuchtungen (LAA, LAB) die Strahlung einer jeden Lichtquelle (A, B) in Bezug auf die anderen Lichtquellen einen Phasenversatz von 360 °/n aufweist und/oder die Strahlung einer jeden Lichtquelle eine Frequenz v/n mit v = Detektionsfrequenz aufweist.

14. Verfahren nach zumindest einem der vorhergehenden Ansprüche,
**dadurch gekennzeichnet,**
**dass** durch die Änderung der Phasenlage aus dem Signal eines Pixels (PI1) ermittelt wird, ob der Messpunkt auf dem Objekt (O) in, auf oder unterhalb der Fokusebene liegt.

15. Verfahren nach zumindest einem der vorhergehenden Ansprüche,
**dadurch gekennzeichnet,**
**dass** aus der Art des Übersprechens der von den einander zugeordneten ersten und zweiten Beleuchtungspunkten (BPA, BPB) erzeugten Messsignale die Konturdaten des Objektes (O) ermittelt werden.

16. Verfahren nach zumindest einem der vorhergehenden Ansprüche,
**dadurch gekennzeichnet,**
**dass** die Messsignale zur Ermittlung optischer Eigenschaften spektral ausgewertet werden.

17. Anordnung zur Erfassung von Konturdaten eines dreidimensionalen Objektes (O), insbesondere eines semitransparenten Objektes wie zahnmedizinischen Objekts, umfassend
- zumindest zwei Multipunktbeleuchtungen (LAA, LAB), sowie einen Pixel (PI1, PI2) aufweisenden Sensor (S),
wobei zwischen den Multipunktbeleuchtungen (LAA, LAB) und dem Objekt (O) und diesem und dem Sensor eine optische Einrichtung, vorzugsweise für konfokale, OCT- oder depth of focus-Strahlengänge angeordnet wird, über die zum einen von den Multipunktbeleuchtungen (LAA, LAB) voneinander versetzte Raster von Beleuchtungspunkten (BRA, BRB) auf das Objekt (O) projiziert und zum anderen die Beleuchtungspunkte (BRA, BRB) auf den Sensor (S) rückprojiziert werden,
- wobei der Abstand zweier Pixel (PI1, PI2) kleiner ist als der Abstand (E) zwischen den auf den Sensor (S) rückprojizierten Beleuchtungspunkten eines Beleuchtungsrasters (BRA, BRB) und somit auch als der Abstand (D) zwischen den auf den Sensor (S) rückprojizierten, einander zugeordneten ersten und zweiten Beleuchtungspunkten (BPA, BPB) der zueinander versetzten Beleuchtungsraster (BRA, BRB), wobei die ersten Beleuchtungspunkte (BPA) von einer ersten und die zweiten Beleuchtungspunkte (BPB) von einer zweiten der zumindest zwei Multipunktbeleuchtungen (LAA, LAB) stammen,
die Anordnung weiterhin umfassend
- Mittel zur Intensitätsmodulation der Strahlungen der Beleuchtungspunkte (BPA, BPB) der Multipunktbeleuchtungen (LAA, LAB) und
- Mittel zur frequenz- und/oder phasenselektiven Detektion der auf den Sensor (S) rückprojizierten, einander zugeordneten ersten und zweiten Beleuchtungspunkte (BPA, BPB),
- wobei der Abstand (D) der ersten und zweiten Beleuchtungspunkte relativ zum Pixelabstand so ist, dass deren frequenz- und/oder phasenselektive Detektion es erlaubt, aus einer Auswertung der Unterschiede von Intensität und/oder Frequenz der Messsignale benachbarter Pixel (PI1, PI2) des Sensors (S) Konturdaten zu ermitteln.

18. Anordnung nach Anspruch 17,
**dadurch gekennzeichnet,**
**dass** die Multipunktbeleuchtungen (LAA, LAB) kollinear angeordnet sind.

19. Anordnung nach Anspruch 17,
**dadurch gekennzeichnet,**
**dass** die Multipunktbeleuchtungen (LAA, LAB) derart zueinander angeordnet sind, dass die die Beleuchtungspunkte (BPA, BPB) bildenden Strahlen geneigt zueinander verlaufen.

20. Anordnung nach zumindest einem der Ansprüche 17 bis 19,
**dadurch gekennzeichnet,**
**dass** der Abstand zwischen den Beleuchtungspunkten (BPA, BPB) in der Fokusebene eines jeden Beleuchtungsrasters (BRA, BRB) zwischen 100 µm und 300 µm liegt, betrachtet zwischen Beleuchtungspunktmitte zu Beleuchtungspunktmitte.

21. Anordnung nach zumindest einem der Ansprüche 17 bis 20,
**dadurch gekennzeichnet,**
**dass** Abstand der Beleuchtungspunkte eines jeden Beleuchtungsrasters (BRA, BRB) in der Fokusebene dreimal bis fünfzehnmal größer als Abstand zwischen benachbarter einander zugeordneter Beleuchtungspunkte (BPA, BPB) der Beleuchtungsraster (BRA, BRB) in der Fokusebene ist.

22. Anordnung nach zumindest einem der Ansprüche 17 bis 21,
**dadurch gekennzeichnet,**
**dass** Abstand benachbarter Pixel (PI1, PI2) des Sensors (S) gleich oder größer als Abstand zweier einander zugeordneter Beleuchtungspunkte (BPA, BPB) der Beleuchtungsraster (BRA, BRB) in der Fokusebene ist.

## Claims

1. Method for acquisition of contour data of a three-dimensional object (O), in particular of a semitransparent object, such as a dental object, wherein by means of an optical device, preferably for confocal or OCT or depth of focus beam paths, offset grids of illumination points (BPA, BPB) of at least two multi-point illuminations (LAA, LAB) are projected onto the object (O) and these are then projected back onto a sensor (S) which has pixels (PI1, PI2), wherein the spacing of two pixels (PI1, PI2) is smaller than the spacing (E) between the illumination points of an illumination grid (BRA, BRB) that are projected back onto the sensor (S) and, thus, also smaller than the spacing (D) between the first and second illumination points (BPA, BPB) of the offset illumination grids (BRA, BRB), said illumination points are projected back onto the sensor (S) and are assigned to each other, wherein the first illumination points (BPA) originate from a first and the second illumination points (BPB) originate from a second multi-point illumination of the at least two multi-point illuminations (LAA, LAB),
wherein the radiations of the illumination points (BPA, BPB) of the multi-point illuminations (LAA, LAB) are modulated in their intensities, and a frequency- and/or phase-selective detection of the first and second reciprocally assigned illumination points (BPA, BPB) that are projected back to the sensor (S) takes place, and that
for the determination of contour data differences of intensity and/or frequency of the measuring signals of adjacent pixels (PI1, PI2) of the sensor (S) are evaluated.

2. Method according to claim 1,
**characterized in that**
a first and a second multi-point illumination (LAA, LAB) are used, which respectively image an illumination point grid (BRA, BRB) onto the object, in which the spacing of the reciprocally assigned first and second illumination points is smaller than the spacing of the first and second illumination points (BPA, BPB), respectively, in the respective pixel grid in the focal plane.

3. Method according to claim 1 or 2,
**characterized in that**
the spacing between adjacent first illumination points and adjacent second illumination points, respectively, in the focal plane is selected at least three times larger than the spacing between a first illumination point (BPA) and a second illumination point (BPB) that is assigned to it.

4. Method according to at least one of the preceding claims,
**characterized in that**
the spacing of adjacent first and second illumination points (BPA, BPB), respectively, when viewed between their center points in the focal plane is determined such that it is approximately between 100 µm and 300 µm.

5. Method according to at least one of the preceding claims,
**characterized in that**
the modulation of the radiation and radiations, respectively, and the imaging of the illumination points (BPA, BPB) onto the object (O) occurs such that a spatial and/or chronologically changing intensity distribution in the immediate local vicinity of at least one pixel (PI1, PI2) of the image sensor (S) occurs.

6. Method according to at least one of the preceding claims,
**characterized in that**
the radiations of the at least two multi-point illuminations (LAA, LAB) are modulated anti-phase and that for the determination of the contour of the object (O) the maximum signal difference between adjacent pixels (PI1, PI2) of the image sensor (S) are evaluated, in the area of said pixels each a first illumination point and a second illumination point (BPA, BPB) that is assigned to said first illumination point are imaged.

7. Method according to at least one of the preceding claims,
**characterized in that**
the at least two multi-point illuminations (LAA, LAB) are arranged collinear.

8. Method according to at least one of the preceding claims,
**characterized in that**
the beams creating the first and second illumination points (BPA, BPB) of the at least two multi-point illuminations (LAA, LAB) are running inclined and at an angle to one another, respectively.

9. Method according to claim 8,
**characterized in that**
at least one of the at least two multi-point illuminations (LAA, LAB) runs inclined to the direction of the measuring beam (MR).

10. Method according to claim 8,
**characterized in that**
the at least two multi-point illuminations (LAA, LAB) are arranged reciprocally angled in relation to the direction of the measuring beam (MR).

11. Method according to at least one of the preceding claims,
**characterized in that**
with multi-point illuminations (LAA, LAB) the beams of which that impinge on the object (O) run at an angle to each other, an optic is used which is configured weakly focusing or not focusing.

12. Method according to at least one of the preceding claims,
**characterized in that**
the spacing between a pair of reciprocally assigned first and second illumination points (BPA, BPB) to the spacing of the pixels (PI1, PI2) of the sensor (S) is selected such that with measurements in the focal plane the first illumination points (BPA) are impinging on pixel-free areas of the image sensor (S) and the second illumination points (BPB) impinge on a pixel (PM) of the image sensor (S) and that during defocusing both the first as well as the second illumination points impinge on pixels (PI1, PI2).

13. Method according to at least one of the preceding claims,
**characterized in that**
during use of n-light sources for generating corresponding multi-point illuminations (LAA, LAB), the radiation of each light source (A, B) with reference to the other light sources has a phase shift of 360°/n and/or the radiation of each light source has a frequency v/n with v = detection frequency.

14. Method according to at least one of the preceding claims,
**characterized in that**
by changing the phase relation it is determined from the signal of a pixel (PI1) whether the measuring point on the object (O) is in, on, or below the focal plane.

15. Method according to at least one of the preceding claims,
**characterized in that**
the contour data of the object (O) are determined from the type of the crosstalk of the measuring signals created from the reciprocally assigned first and second illumination points (BPA, BPB).

16. Method according to at least one of the preceding claims,
**characterized in that**
the measuring signals for the determination of optical characteristics are spectrally evaluated.

17. Arrangement for acquisition of contour data of a three-dimensional object (O), in particular of a semitransparent object, such as a dental object, comprising
at least two multi-point illuminations (LAA, LAB) as well as
a sensor (S) comprising pixels (PI1, PI2),
wherein between the multi-point illuminations (LAA, LAB) and the object (O) and said object and the sensor an optical device is arranged, preferably for confocal, OCT or depth of focus beam paths, by means of which, on the one hand, offset grids of illumination points (BRA, BRB) are projected from the multi-point illuminations (LAA, LAB) onto the object (O), and on the other hand, the illumination points (BRA, BRB) are projected back onto the sensor (S), wherein the spacing of two pixels (PI1, PI2) is smaller than the spacing (E) between the illumination points of an illumination grid (BRA, BRB) which are projected back onto the sensor (S) and, thus, also smaller than the spacing (D) between the first and second illumination points (BPA, BPB) of the offset illumination grids (BRA, BRB), said illumination points are projected back onto the sensor (S) and are assigned to each other and, wherein the first illumination points (BPA) originate from a first and the second illumination points (BPB) originate from a second multi-point illumination of the at least two multi-point illuminations (LAA, LAB),
the arrangement further comprising
- means for the intensity modulation of the radiations of the illumination points (BPA, BPB) of the multi-point illuminations (LAA, LAB), and
- means for the frequency- and/or phase-selective detection of the first and second illumination points (BPA, BPB) which are assigned to each other and projected back to the sensor (S),
- wherein the spacing (D) of the first and second illumination points relative to the pixel spacing is so that their frequency- and/or phase-selective detection allows to determine contour data from an evaluation of the differences of intensity and/or frequency of the measurement signals of adjacent pixels (PI1, PI2) of the sensor (S).

18. Arrangement according to claim 17,
**characterized in that**
the multi-point illuminations (LAA, LAB) are arranged collinear.

19. Arrangement according to claim 17,
**characterized in that**
the multi-point illuminations (LAA,LAB) are arranged to each other such that the beams that are creating the illumination points (BPA, BPB) run inclined towards each other.

20. Arrangement according to at least one of the claims 17 to 19,
**characterized in that**
the spacing between the illumination points (BPA, BPB) in the focal plane of each illumination grid (BRA, BRB) is between 100 µm and 300 µm, when viewed between illumination point center to illumination point center.

21. Arrangement according to at least one of the claims 17 to 20,
**characterized in that**
spacing of the illumination points of each illumination grid (BRA, BRB) in the focal plane is 3 times up to 15 times larger than spacing between adjacent reciprocally assigned illumination points (BPA, BPB) of the illumination grids (BRA, BRB) in the focal plane.

22. Arrangement according to at least one of the claims 17 to 21,
**characterized in that**
spacing of adjacent pixels (PI1, PI2) of the sensor (S) is the same as or larger than spacing between two reciprocally assigned illumination points (BPA, BPB) of the illumination grids (BRA, BRB) in the focal plane.

## Revendications

1. Procédé pour l'acquisition de données de contour d'un objet (O) tridimensionnel, en particulier d'un objet semi-transparent tel qu'un objet de médecine dentaire, selon lequel, au moyen d'un dispositif optique, de préférence pour trajectoires de faisceaux à foyer commun ou OCT ou *depth of focus* (profondeur de mise au point), des trames de points lumineux (BPA, BPB) provenant d'au moins deux éclairages multipoints (LAA, LAB) et décalées entre elles sont projetées sur l'objet (O) et celles-ci sont ensuite rétroprojetées sur un capteur (S) disposant de pixels (PI1, PI2),
- sachant que la distance de deux pixels (PI1, PI2) est inférieure à la distance (E) entre les points lumineux d'une trame lumineuse (BRA, BRB) qui sont rétroprojetés sur le capteur (S), et par conséquent également inférieure à la distance (D) entre les premiers et deuxièmes points lumineux (BPA, BPB) des trames lumineuses (BRA, BRB) décalées entre elles qui sont associés entre eux et rétroprojetés sur le capteur (S), les premiers points lumineux (BPA) provenant d'un premier et les deuxièmes points lumineux (BPB) d'un deuxième des au moins deux éclairages multipoints (LAA, LAB),
- sachant que les rayonnements des points lumineux (BPA, BPB) des éclairages multipoints (LAA, LAB) sont modulés dans leurs intensités et qu'est effectuée une détection sélective en fréquences et/ou en phases des premiers et deuxièmes points lumineux (BPA, BPB) associés entre eux rétroprojetés sur le capteur (S) et
- que pour déterminer les données de contour, des différences d'intensité et/ou de fréquence des signaux de mesure de pixels voisins (PI1, PI2) du capteur (S) sont analysées.

2. Procédé selon la revendication 1,
**caractérisé en ce**
**que** sont utilisés un premier et un deuxième éclairages multipoints (LAA, LAB) qui reproduisent chacun sur l'objet une trame de points lumineux (BRA, BRB), dans lesquelles la distance des premiers et deuxièmes points lumineux associés entre eux est inférieure à la distance dans le plan focal des premiers et des deuxièmes points lumineux (BPA, BPB) dans chaque trame de points d'image.

3. Procédé selon la revendication 1 ou 2,
**caractérisé en ce**
**que** la distance entre des premiers points lumineux voisins respectivement deuxièmes points lumineux voisins dans le plan focal est choisie au moins trois fois plus grande que la distance entre un premier point lumineux (BPA) et un deuxième point lumineux (BPB) associé à celui-ci.

4. Procédé selon au moins une des revendications précédentes,
**caractérisé en ce**
**que** la distance de premiers respectivement deuxièmes points lumineux (BPA, BPB) voisins considérée entre leurs centres dans le plan focal est fixée de manière telle qu'elle se situe approximativement entre 100 µm et 300 µm.

5. Procédé selon au moins une des revendications précédentes,
**caractérisé en ce**
**que** la modulation du rayonnement respectivement des rayonnements et la reproduction des points lumineux (BPA, BPB) sur l'objet (O) sont effectuées de manière telle qu'a lieu une répartition des intensités se modifiant dans l'espace et/ou dans le temps dans le voisinage local direct d'au moins un pixel (PI1, PI2) du capteur d'image (S).

6. Procédé selon au moins une des revendications précédentes,
**caractérisé en ce**
**que** les rayonnements des au moins deux éclairages multipoints (LAA, LAB) sont modulés en opposition de phase et que, pour déterminer le contour de l'objet (O), est évaluée la différence maximale de signal entre des pixels voisins (PI1, PI2) du capteur d'image (S), dans la zone desquels sont respectivement reproduits un premier et un deuxième points lumineux (BPA, BPB), ce dernier étant associés au premier.

7. Procédé selon au moins une des revendications précédentes,
**caractérisé en ce**
**que** les au moins deux éclairages multipoints (LAA, LAB) sont disposés de manière colinéaire.

8. Procédé selon au moins une des revendications précédentes,
**caractérisé en ce**
**que** les faisceaux des au moins deux éclairages multipoints (LAA, LAB) générant les premiers et deuxièmes points lumineux (BPA, BPB) s'étendent de manière inclinée ou en faisant un angle entre eux.

9. Procédé selon la revendication 8,
**caractérisé en ce**
**qu'**au moins l'un des au moins deux éclairages multipoints (LAA, LAB) s'étend de manière inclinée par rapport à la direction du faisceau de mesure (MR).

10. Procédé selon la revendication 8,
**caractérisé en ce**
**que** par rapport à la direction du faisceau de mesure (MR), les au moins deux éclairages multipoints (LAA, LAB) sont disposés en faisant un angle entre eux.

11. Procédé selon au moins une des revendications précédentes,
**caractérisé en ce**
**que** pour les éclairages multipoints (LAA, LAB) dont les faisceaux se propageant en faisant un angle entre eux rencontrent l'objet (O), est utilisé un instrument optique qui est conçu de manière faiblement focalisante ou pas focalisante.

12. Procédé selon au moins une des revendications précédentes,
**caractérisé en ce**
**que** la distance entre une paire de premiers et deuxièmes points lumineux (BPA, BPB) associés entre eux relativement à la distance des pixels (PI1, PI2) du capteur (S) est choisie de manière telle que, lors des mesures dans le plan focal, les premiers points lumineux (BPA) rencontrent des zones exemptes de pixels du capteur d'image (S) et les deuxièmes points lumineux (BPB) rencontrent un pixel (PM) du capteur d'image (S), et que lors de la défocalisation, les premiers ainsi que les deuxièmes points lumineux rencontrent des pixels (PI1, PI2).

13. Procédé selon au moins une des revendications précédentes,
**caractérisé en ce**
**que** lors de l'utilisation de n sources lumineuses pour créer des éclairages multipoints (LAA, LAB) correspondants, le rayonnement de chaque source lumineuse (A, B) présente un décalage de phase de 360°/n par rapport aux autres sources lumineuses et/ou le rayonnement de chaque source lumineuse présente une fréquence v/n où v = fréquence de détection.

14. Procédé selon au moins une des revendications précédentes,
**caractérisé en ce**
**que** par la modification de la position de phase à partir du signal d'un pixel (PI1), il est déterminé si le point de mesure sur l'objet (O) est situé dans, sur ou sous le plan focal.

15. Procédé selon au moins une des revendications précédentes,
**caractérisé en ce**
**que** les données de contour de l'objet (O) sont déterminées à partir de la nature du couplage croisé des signaux de mesure des premiers et deuxièmes points lumineux (BPA, BPB) associés entre eux.

16. Procédé selon au moins une des revendications précédentes,
**caractérisé en ce**
**que** les signaux de mesure sont analysés spectralement pour déterminer des propriétés optiques.

17. Dispositif pour l'acquisition de données de contour et/ou de propriétés optiques d'un objet tridimensionnel (O), en particulier d'un objet semi-transparent tel qu'un objet de médecine dentaire, ledit dispositif comprenant
- au moins deux éclairages multipoints (LAA, LAB), ainsi qu'un capteur (S) disposant de pixels (PI1, PI2),
sachant qu'entre les éclairages multipoints (LAA, LAB) et l'objet (O) et ledit objet et le capteur est disposé un dispositif optique, de préférence pour trajectoires de faisceaux à foyer commun ou OCT ou *depth of focus* (profondeur de mise au point), au moyen duquel sont d'une part projetées sur l'objet (O) des trames de points lumineux (BRA, BRB) décalées entre elles provenant des éclairages multipoints (LAA, LAB), et sont d'autre part rétroprojetés sur le capteur (S) les points lumineux (BRA, BRB),
- sachant que la distance de deux pixels (PI1, PI2) est inférieure à la distance (E) entre les points lumineux d'une trame lumineuse (BRA, BRB) qui sont rétroprojetés sur le capteur (S), et par conséquent également inférieure à la distance (D) entre les premiers et deuxièmes points lumineux (BPA, BPB) des trames lumineuses (BRA, BRB) décalées entre elles qui sont associés entre eux et rétroprojetés sur le capteur (S), les premiers points lumineux (BPA) provenant d'un premier et les deuxième points lumineux (BPB) d'un deuxième des au moins deux éclairages multipoints (LAA, LAB),
le dispositif comprenant en outre
- des moyens de modulation des rayonnements des points lumineux (BPA, BPB) des éclairages multipoints (LAA, LAB) et
- des moyens de détection sélective en fréquences et/ou en phases des premiers et deuxièmes points lumineux (BPA, BPB) associés entre eux rétroprojetés sur le capteur (S),
- sachant que la distance (D) des premiers et deuxièmes points lumineux relativement à la distance des pixels est telle que leur détection sélective en fréquences et/ou en phases permet de déterminer des données de contour à partir d'une analyse des différences d'intensité et/ou de fréquence des signaux de mesure de pixels voisins (PI1, PI2) du capteur (S).

18. Dispositif selon la revendication 17,
**caractérisé en ce**
**que** les éclairages multipoints (LAA, LAB) sont disposés de manière colinéaire.

19. Dispositif selon la revendication 17,
**caractérisé en ce**
**que** les éclairages multipoints (LAA, LAB) sont disposés les uns par rapport aux autres de sorte que les faisceaux formant les points lumineux (BPA, BPB) s'étendent de manière inclinée entre eux.

20. Dispositif selon au moins une des revendications 17 à 19,
**caractérisé en ce**
**que** la distance entre les points lumineux (BPA, BPB) dans le plan focal de chaque trame lumineuse (BRA, BRB) se situe entre 100 µm et 300 µm, considérée de centre de point lumineux à centre de point lumineux.

21. Dispositif selon au moins une des revendications 17 à 20,
**caractérisé en ce**
**que** la distance dans le plan focal des points lumineux de chaque trame lumineuse (BRA, BRB) est trois à quinze fois plus grande que la distance dans le plan focal entre des points lumineux (BPA, BPB) voisins associés entre eux de la trame lumineuse (BRA, BRB).

22. Dispositif selon au moins une des revendications 17 à 21,
**caractérisé en ce**
**que** la distance de pixels voisins (PI1, PI2) du capteur (S) est égale ou supérieure à la distance dans le plan focal de deux points lumineux (BPA, BPB) associés entre eux de la trame lumineuse (BRA, BRB).
